# EUROPEAN PATENT APPLICATION

(11) **EP 2 924 428 A1**
(43) Date of publication of application: **30.09.2015**
(21) Application number: 15160268.7
(22) Date of filing: 23.03.2015
(51) Int. Cl.: G01N 29/22, A61B 8/00

(54) **ULTRASONIC MEASURING DEVICE**

(30) Priority: 25.03.2014 JP 2014061552
(71) Applicant: Seiko Epson Corporation, Shinjuku-ku, Tokyo 163-0811 (JP)
(72) Inventor: Atsuchi, Robina, Nagano, 392-8502 (JP)
(74) Representative: Hoffmann Eitle

(57) **Abstract**

An ultrasonic measuring device includes a mounting member, a support unit, a plurality of ultrasonic element array units, a plurality of actuators, and a control unit. The mounting member is configured and arranged to be mounted on a test subject. The support unit is fixed on the mounting member. The ultrasonic element array units are supported on the support unit. The actuators are operatively coupled respectively to the ultrasonic element array units. Each of the actuators is configured and arranged to individually displace a corresponding one of the ultrasonic element array units with respect to the support unit. The control unit is configured to control the ultrasonic element array units to transmit and receive ultrasonic waves, the control unit being further configured to control the actuators.

## Description

### BACKGROUND

### Technical Field

The present invention relates to an ultrasonic measuring device and the like.

### Related Art

Japanese Unexamined Patent Publication No. 2004-187885 discloses an arteriosclerosis evaluation device equipped with an ultrasonic probe. The ultrasonic probe of this arteriosclerosis evaluation device is attached to the wrist of the person being tested with a band, and pressed on the skin of the person being tested by adjusting the air pressuring from an air pump, and an ultrasonic image of an artery is detected.

### SUMMARY

Typically, with an ultrasonic probe in which one ultrasonic element array is incorporated, the scanning range of the ultrasonic waves emitted from the ultrasonic element array is limited, so an ultrasonic image of the test subject can only be obtained in a relatively narrow range. On the other hand, with testing and diagnosis, there is a desire to acquire a broader range ultrasonic cross section image of the test subject. In light of that, skillful work was required by the measurer of suitably holding and moving the ultrasonic probe orientation in relation to the test subject. With the ultrasonic probe of Japanese Unexamined Patent Publication No. 2004-187885, it is possible to change the measuring position using a motor drive, but the possible movement range is narrow, and it was difficult to obtain a so-called panoramic image which is an ultrasonic cross section image of a broad range of the test subject formed based on the information of a plurality of ultrasonic cross section images.

Taking into consideration these circumstances, there is demand for an ultrasonic measuring device that contributes to the realization of imaging that can form a panoramic image with as broad a scope as possible.

An ultrasonic measuring device according to one aspect includes a mounting member, a support unit, a plurality of ultrasonic element array units, a plurality of actuators, and a control unit. The mounting member is configured and arranged to be mounted on a test subject. The support unit is fixed on the mounting member. The ultrasonic element array units are supported on the support unit. The actuators are operatively coupled respectively to the ultrasonic element array units. Each of the actuators is configured and arranged to individually displace a corresponding one of the ultrasonic element array units with respect to the support unit. The control unit is configured to control the ultrasonic element array units to transmit and receive ultrasonic waves, the control unit being further configured to control the actuators.

It is possible to have a plurality of the ultrasonic array units mounted on the test subject. The actuators can control the mutual relative positional relationship between the plurality of ultrasonic element array units. In this way, the ultrasonic image imaged with the individual ultrasonic element array units can be combined on one plane (cross section). This kind of ultrasonic measuring device can greatly contribute to the realization of imaging for which it is possible to form a broad range panoramic image along a plane as precisely as possible.

Each of the actuators is preferably configured and arranged to generate a drive force on the corresponding one of the ultrasonic element array units in a direction orthogonal to an array surface of the corresponding one of the ultrasonic element array units. The ultrasonic element array unit is equipped with ultrasonic transducer elements arranged in an array form, and the element surface which is the ultrasonic wave emission surface of those ultrasonic transducer elements is arranged so as to be positioned on the array surface which is a designated plane. The ultrasonic element array unit can be displaced in the direction orthogonal to the array surface of the ultrasonic element array unit according to the drive force of the actuator. As a result, it is possible to have the ultrasonic element array unit press against the test subject with a suitable pressure.

Each of the actuators is preferably configured and arranged to generate a drive force on the corresponding one of the ultrasonic element array units around at least one of an x axis and a y axis in a two dimensional coordinate system parallel to an array surface of the corresponding one of the ultrasonic element array units. The orientation of the ultrasonic element array unit can change around at least one of the x axis and the y axis parallel to the array surface of the ultrasonic element array unit according to the drive force of the actuator. As a result, it is possible to have the ultrasonic element array unit press against the test subject with a suitable orientation.

Each of the actuators is preferably configured and arranged to generate a drive force on the corresponding one of the ultrasonic element array units around both of the x axis and the y axis in the two dimensional coordinate system. The orientation of the ultrasonic element array unit can change around both of the x axis and the y axis parallel to the array surface of the ultrasonic element according to the drive force of the actuator. As a result, it is possible to have the ultrasonic element array unit press against the test subject with a suitable orientation.

The control unit is preferably configured to control the actuators to change a pressing pressure of the ultrasonic element array units on the test subject so that the ultrasonic element array units transmit receive the ultrasonic waves at a first pressure value and at a second pressure value that is different from the first pressure value. It is possible to realize ultrasonic elastography based on the received ultrasonic waves of the first pressure value and the received ultrasonic waves of the second pressure value.

The control unit is preferably configured to control the ultrasonic element array units to transmit and receive surface acoustic waves between adjacent ones of the ultrasonic element array units. It is possible for the control unit to measure the distance between adjacent ultrasonic element array units based on surface acoustic waves that propagate in the surface of the test subject. It is possible to adjust the distance between adjacent ultrasonic element array units based on the measured distance.

The control unit, when pressing the ultrasonic element array units on the test subject, is preferably configured to control the actuators change orientation of the ultrasonic element array units with respect to the support unit based on a distance measured by transmission and reception of the surface acoustic waves. The minimum distance is maintained between the adjacent ultrasonic element array units according to changes in the orientation of this kind of ultrasonic element unit array. As a result, it is possible to protect the site of the person being tested, for example, from being pinched between adjacent ultrasonic element array units.

The control unit is preferably configured to control one of the actuators to release a pressing pressure of the corresponding one of the ultrasonic element array units on the test subject, and to change orientation of the corresponding one of the ultrasonic element array units around at least one of an x axis and a y axis in a two dimensional coordinate system parallel to an array surface of the corresponding one of the ultrasonic element array units. The minimum distance is maintained between adjacent ultrasonic element array units according to changes in the orientation of this kind of ultrasonic element array unit. As a result, it is possible to protect the site of the person being tested, for example, from being pinched between adjacent ultrasonic element array units.

The control unit is preferably configured to control the one of the actuators to change the orientation of the corresponding one of the ultrasonic element array units around bot of the x axis and the y axis in the two dimensional coordinate system. The minimum distance is maintained between adjacent ultrasonic element array units according to changes in the orientation of this kind of ultrasonic element array unit. As a result, it is possible to protect the site of the person being tested, for example, from being pinched between adjacent ultrasonic element array units.

### BRIEF DESCRIPTION OF THE DRAWINGS

Referring now to the attached drawings which form a part of this original disclosure:
FIG. 1 is a block diagram schematically showing the constitution of an ultrasonic device as electronic equipment with one embodiment.
FIG. 2 is a flow chart schematically showing the operation of the ultrasonic diagnostic device.
FIG. 3 is a pattern diagram schematically showing the panoramic image concept.
FIG. 4 is a graph showing the changes in pressing pressure applied when implementing ultrasonic elastography.

### DETAILED DESCRIPTION OF EXEMPLARY EMBODIMENTS

Following, an embodiment of the present invention while referring to the attached drawings will be described. This embodiment described below is not unreasonably limited to the contents of the present invention noted in the claims, and is not limited to necessarily having all the constitutions described with this embodiment as the means of solving the present invention.

### (1) Constitution of Ultrasonic Diagnostic Device

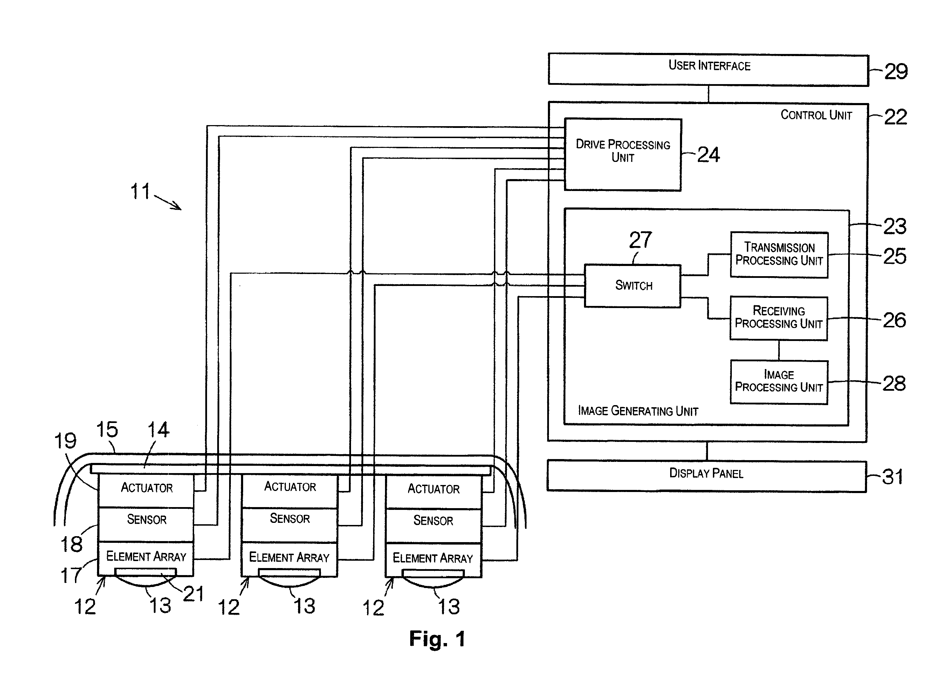

FIG. 1 schematically shows the constitution of the ultrasonic diagnostic device (ultrasonic measuring device) 11 as electronic equipment of one embodiment. The ultrasonic diagnostic device 11 is equipped with a plurality of ultrasonic assemblies 12. The ultrasonic assembly 12 has an acoustic lens 13. Ultrasonic waves are transmitted and received through the acoustic lens 13. The ultrasonic assembly 12 is linked to a support unit 14. The support unit 14 is formed from a plate material or the like, for example. The support unit 14 is fixed to a belt 15 as a mounting means. The belt 15 can be mounted on a test subject. The ultrasonic assembly 12 contacts the test subject (person being tested) with the acoustic lens 13. The acoustic lens 13 has a partial cylindrical surface on which a generatrix is formed parallel to the center axis. The partial cylindrical surface determines the focal position of the ultrasonic waves. The ultrasonic assemblies 12 are arrayed in one row so that the generatrixes of the acoustic lenses 13 align in series. The support unit 14 can have rigidity to the level that it is not deformed when mounted on the test subject, or can have flexibility to the level that it follows the surface of the test subject.

The individual ultrasonic assemblies 12 are formed from an ultrasonic element array unit 17, a sensor unit (sensor) 18, and an actuator unit (actuator) 19. The ultrasonic element array unit 17 is equipped with an ultrasonic device 21. The ultrasonic device 21 has ultrasonic transducer elements arranged in array form on a substrate. Ultrasonic waves are transmitted to and received from each individual ultrasonic element. The acoustic lenses 13 are covered on the array of the ultrasonic transducer elements. Here, a three dimensional coordinate system is fixed to the array surface of the ultrasonic element array unit 17. An xy plane of the three dimensional coordinate system is overlapped on the array surface of the ultrasonic element array unit 17. The x axis correlates to the intersecting line of the perpendicular surface that extends perpendicularly to the xy plane from the center axis of the acoustic lens 13 and the xy plane. The y axis correlates to the intersecting line of the plane that bisects the cylindrical surface of the acoustic lens 13 that crosses at a right angle the generatrix of the acoustic lens 13 and the xy plane. The z axis is defined in the direction orthogonal to the array surface of the ultrasonic element array unit 17. The array surface of the ultrasonic element array unit 17 matches the substrate surface of the ultrasonic device 21.

The sensor unit 18 is equipped with for example pressure sensors and orientation detection sensors. It detects pressure that acts on the ultrasonic element array unit 17 from the support unit 14 in the direction (z axis direction) orthogonal to the array surface of the ultrasonic element array unit 17. The orientation detection sensor detects the rotation angle of the ultrasonic element array unit 17 around the x axis and around the y axis with the two dimensional coordinate system parallel to the array surface of the ultrasonic element array unit 17. The orientation of the ultrasonic element array unit 17 is specified based on these rotation angles. Here, it is possible to use a gyro sensor for the orientation detection sensor.

The actuator unit 19 links the ultrasonic element array unit 17 to the support unit 14. The actuator unit 19 is equipped with a pressure drive unit, an x axis rotation drive unit, and a y axis rotation drive unit, for example. The pressure drive unit generates drive force on the ultrasonic element array unit 17 in the z axis direction. The ultrasonic element array unit 17 is displaced in the z axis direction according to the drive force generated by the pressure drive unit. It is possible to control the pressing pressure of the ultrasonic element array unit 17 on the test subject according to the displacement in the z axis direction. The x axis rotation drive unit generates drive force on the ultrasonic element array unit 17 around the x axis with the two dimensional coordinate system parallel to the array surface of the ultrasonic element array unit 17. The y axis rotation drive unit generates drive force on the ultrasonic element array unit 17 around the y axis with the two dimensional coordinate system parallel to the array surface of the ultrasonic element array unit 17. The orientation of the ultrasonic element array unit 17 changes around the x axis or around the y axis according to the drive force generated by the x axis rotation drive unit or the y axis rotation drive unit. For the pressure drive unit, the x axis rotation drive unit, and the y axis rotation drive unit, it is possible to use an ultrasonic motor having a high holding power, for example. However, instead of the ultrasonic motor, it is also possible to use another drive mechanism including an electric motor.

A control unit 22 is connected to the ultrasonic assemblies 12. Here, one control unit 22 is incorporated in common to the plurality of ultrasonic assemblies 12. The control unit 22 is equipped with an image generating unit 23 and a drive processing unit 24. The image generating unit 23 is connected to the ultrasonic element array unit 17 for each individual ultrasonic assembly 12. The image generating unit 23 controls transmission and receiving of ultrasonic waves. The drive processing unit 24 is connected to the sensor unit 18 and the actuator unit 19 for each individual ultrasonic assembly 12. The drive processing unit 24 controls the operation of the actuator unit 19 according to the output of the sensor unit 18 for each individual ultrasonic assembly 12.

The image generating unit 23 is equipped with a transmission processing unit 25 and a receiving processing unit 26. The transmission processing unit 25 and a receiving processing unit 26 are connected to each individual ultrasonic assembly 12 via a switch 27. The switch 27 switches between the connection of the transmission processing unit 25 and the connection of the receiving processing unit 26 for each individual ultrasonic assembly 12. Oscillation signals are supplied to each individual ultrasonic element array unit 17 from the transmission processing unit 25. Ultrasonic waves are emitted from each individual ultrasonic element array unit 17 according to the supply of oscillation signals. Ultrasonic wave received signals are generated by the ultrasonic element array unit 17 by the reflected ultrasonic waves. The received signals are transmitted to the receiving processing unit 26. An image processing unit 28 is connected to the receiving processing unit 26. The image processing unit 28 generates an ultrasonic image according to the received signals. The image processing unit 28 is able to generate a so-called panoramic image based on the plurality of images fetched for each ultrasonic assembly 12. With the panoramic image, one ultrasonic image is drawn over a broad range by combining a plurality of tomographic images.

The drive processing unit 24 controls the operation of the pressure drive unit of the actuator unit 19 based on the pressure value of the z axis direction detected by the sensor unit 18. Similarly, the drive processing unit 24 controls the operation of the x axis rotation drive unit of the actuator unit 19 based on the rotation angle around the x axis detected by the sensor unit 18. The drive processing unit 24 controls the operation of the y axis rotation drive unit of the actuator unit 19 based on the rotation angle around the y axis detected by the sensor 18.

A user interface 29 and a display panel 31 are connected to the control unit 22. The user interface 29 can be, for example, a touch screen panel, keyboard, voice recognition device or the like. The user of the ultrasonic diagnostic device 11 can input instructions to the control unit 22 through the user interface 29. The user operates the ultrasonic diagnostic device 11 by inputting instructions to the control unit 22. For the display panel 31, it is possible to use a flat display such as a liquid crystal panel or an organic EL (electroluminescence) panel, for example. Ultrasonic images are projected onto the display panel 31 based on the image signals supplied from the image processing unit 28. In addition, it is also possible to display text information or icons on the display panel 31.

### (2) Operation of Ultrasonic Diagnostic Device

Next, a brief description of the operation of the ultrasonic diagnostic device 11 will be provided. The ultrasonic assembly 12 is mounted on the person being tested. The belt 15 is wound onto the arm, abdomen or the like. With each individual ultrasonic assembly 12, the acoustic lens 13 is pressed on the skin of the person being tested, for example. Before mounting, the position and orientation of each individual ultrasonic assembly 12 is specified within the overall spatial coordinate system. For example, the generatrix of the acoustic lens 13 is aligned in series at equal intervals. This initial position and initial orientation are stored as the reference position and the reference orientation.

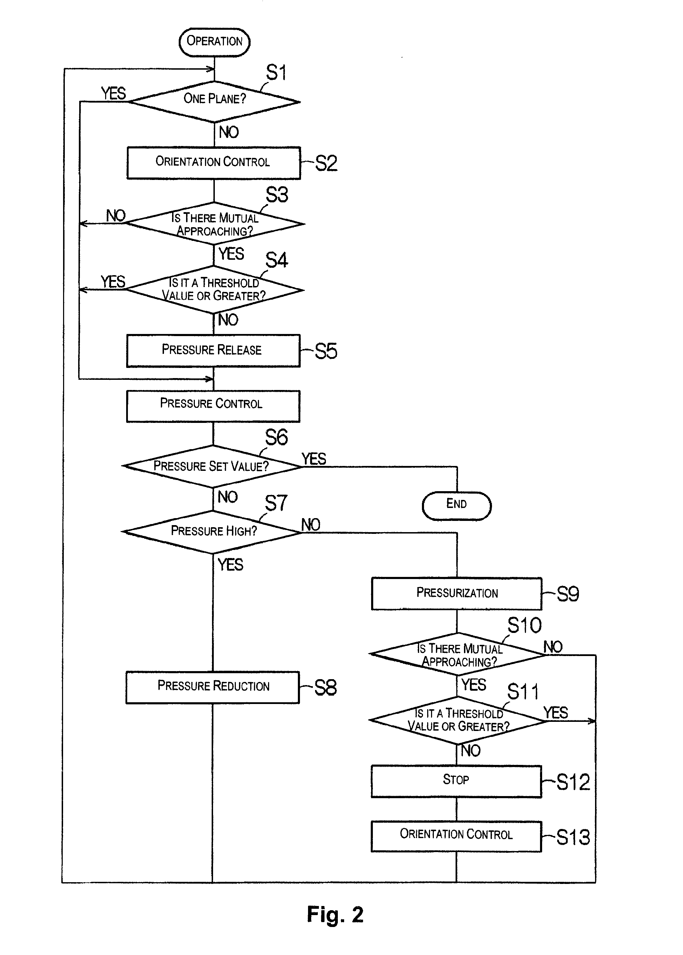

As shown in FIG. 2, the position and orientation of the ultrasonic element array unit 17 are detected individually by the drive processing unit 24 at the time of mounting. The displacement and changes in relation to the reference position and the reference orientation are specified. At step S1, a judgment is made of whether or not the cross section surface of each individual ultrasonic assembly 12 overlaps one plane. If overlapping, the operation of the drive processing unit 24 shifts to pressure control. If not overlapping, at step S2, the drive processing unit 24 implements orientation control. The drive processing unit 24 outputs drive signals to the x axis rotation drive unit and the y axis rotation drive unit based on the rotation angle around the x axis and the rotation angle around the y axis specified by the sensor unit 18. Drive force is generated around the x axis and around the y axis according to the drive signals supplied by the x axis rotation drive unit and the y axis rotation drive unit.

With implementation of the orientation control, at step S3, the drive processing unit 24 determines whether or not there is mutual approaching between adjacent ultrasonic element array units 17. If there is no mutual approaching, the drive processing unit 24 operation shifts to pressure control. If mutual approaching is found, at step S4, a judgment is made of whether or not the gap between adjacent ultrasonic element array units 17 is a threshold value or greater. If it is the threshold value or greater, the drive processing unit 24 operation shifts to pressure control. If it is less than the threshold value, the drive processing unit 24, at step S5, releases the drive force (or holding force) of the pressure drive unit. In this way, the ultrasonic element array unit 17 is able to distance itself from the test subject. As a result, with adjacent ultrasonic element array units 17, the minimum distance is maintained. It is possible to protect the skin of the person being tested from being pinched between adjacent ultrasonic element array units 17. When the orientation control is completed, the drive processing unit 24 operation shifts to pressure control.

With the pressure control, the drive processing unit 24 detects pressing pressure by the ultrasonic element array units 17 individually. At step S6, a judgment is made of whether or not the pressing pressure is a set value. If it is a set value, the operation of the drive processing unit 24 ends. After that, the ultrasonic image is generated by the work of the image generating unit 23.

If the pressing pressure is not a set value, at step S7, a judgment is made of whether or not the detected pressure value is higher than the set value. If it is higher, than at step S8, pressure reduction processing is implemented. The drive processing unit 24 weakens the drive force of the pressure drive unit of the actuator 19. The pressing pressure of the ultrasonic element array unit 17 is decreased. After that, the drive processing unit 24 operation returns to step S1. Then, the same processing as described previously is repeated.

When the detected pressure value is the set value or less, at step S9, the drive processing unit 24 strengths the drive force of the pressure drive unit of the actuator unit 19. The pressing pressure of the ultrasonic element array unit 17 increases. When the pressurizing control is implemented, at step S10, the drive processing unit 24 determines whether or not there is mutual approaching between the adjacent ultrasonic element array units 17. If there is no mutual approaching, the operation of the drive processing unit 24 returns to step S1 again. The processes described previously are repeated. If mutual approaching is found, at step S11, a judgment is made of whether or not the gap between adjacent ultrasonic element array units 17 is a threshold value or greater. If it is the threshold value or greater, the operation of the drive processing unit 24 similarly returns to step S1. If it is less than the threshold value, the drive processing unit 24 stops the increasing of the pressure at step S12. In this way, greater mutual approaching than that is prevented. As a result, the minimum distance is maintained between the adjacent ultrasonic element array units 17. It is possible to protect the skin of the person being tested from being pinched between adjacent ultrasonic element array units 17. After that, at step S 13, orientation control of the ultrasonic element array units 17 is implemented. The drive processing unit 24 does rotation drive of the ultrasonic element array units 17 around the y axis. As a result, even if they progress toward the test subject, it is possible to avoid further mutual approaching of the adjacent ultrasonic element array units 17 than that. After this kind of orientation control is completed, the operation of the drive processing unit 24 returns to step S1.

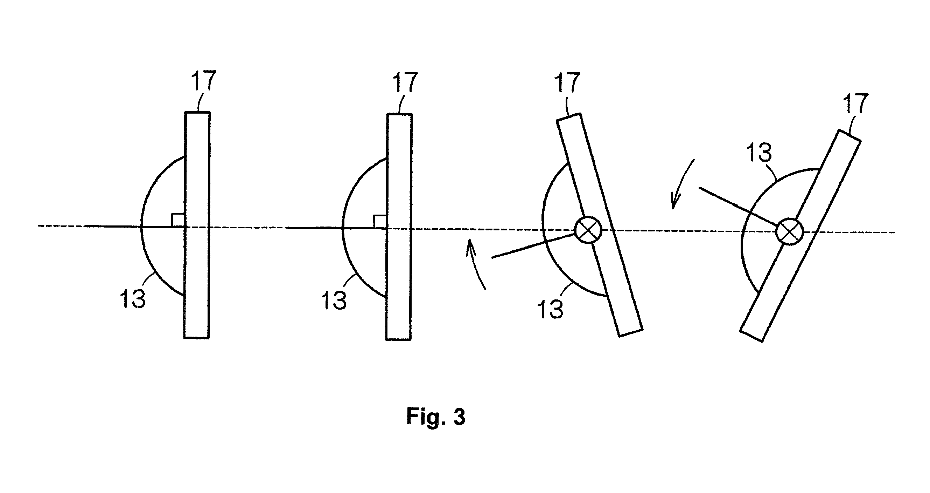

As shown in FIG. 3, it is desirable to have the cross section surface of all the ultrasonic element array units 17 be matched to be on a single plane when generating the panoramic image. With the ultrasonic diagnostic device 11 of this embodiment, it is possible to fix a plurality of ultrasonic element array units 17 to the test subject. The actuator unit 19 is able to control the mutual relative positional relationship of the plurality of ultrasonic element array units 17. The ultrasonic image imaged by the individual ultrasonic element array units in this way can be combined onto one plane. This kind of ultrasonic diagnostic device 11 is able to contribute greatly to the realization of imaging that is able to form a panoramic image along one plane as precisely as possible.

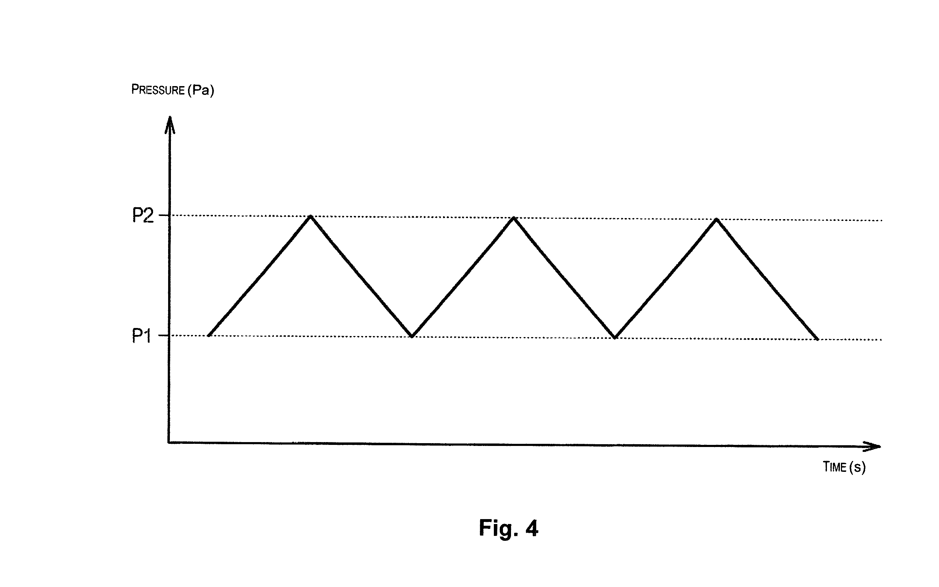

The ultrasonic diagnostic device 11 can be used for implementing ultrasonic elastography. At this time, the drive processing unit 24 controls the pressure drive unit of the actuator unit 19 and changes the pressing pressure of the ultrasonic element array unit 17 on the test subject. For example, as shown in FIG. 4, it is possible to repeat at fixed cycles the variation of the pressure at a constant rising rate and falling rate. The image generating unit 23 fetches the ultrasonic image at a pressure P1 and a pressure P2. The image generating unit 23 detects the relative difference between the pressure P1 image and the pressure P2 image. The comparison results are displayed on the display panel 31.

Surface acoustic waves of the ultrasonic waves can also be used for detection of the previously described mutual approaching. The drive processing unit 24 of the control unit 22 is able to measure the distance between adjacent ultrasonic element array units 17 based on the surface acoustic waves that propagate on the surface of the test subject. The drive processing unit 24 of the control unit 22 changes the orientation of the ultrasonic element array units 17 in relation to the support unit 14 based on the distance measured with transmission and receiving of the surface acoustic waves when pressing the ultrasonic element array units 17 on the test subject. The minimum distance is maintained between adjacent ultrasonic element array units 17 according to this kind of orientation change of the ultrasonic element array units 17. As a result, it is possible to protect the skin of the person being tested from being pinched between adjacent ultrasonic element array units 17, for example.

We gave a detailed description of the embodiment as noted above, but a person skilled in the art will easily understand that it is possible to have many modifications without substantially straying from the novel items and effects of the present invention. Therefore, all of these kinds of modification examples are included within the scope of the present invention. For example, for terminology noted at least once together with a different term having a broader or the same meaning in the specification or drawings, that different terminology can be used as a substitute in any location in the specification or drawings. Also, the constitution and operation of the ultrasonic assembly 12, the support unit 14, the ultrasonic element array unit 17, the sensor unit 18, the actuator unit 19 and the like are not limited to the descriptions with this embodiment, and various modifications are possible.

### GENERAL INTERPRETATION OF TERMS

In understanding the scope of the present invention, the term "comprising" and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms, "including", "having" and their derivatives. Also, the terms "part," "section," "portion," "member" or "element" when used in the singular can have the dual meaning of a single part or a plurality of parts. Finally, terms of degree such as "substantially", "about" and "approximately" as used herein mean a reasonable amount of deviation of the modified term such that the end result is not significantly changed. For example, these terms can be construed as including a deviation of at least ± 5% of the modified term if this deviation would not negate the meaning of the word it modifies.

While only selected embodiments have been chosen to illustrate the present invention, it will be apparent to those skilled in the art from this disclosure that various changes and modifications can be made herein without departing from the scope of the invention as defined in the appended claims. Furthermore, the foregoing descriptions of the embodiments according to the present invention are provided for illustration only, and not for the purpose of limiting the invention as defined by the appended claims and their equivalents.

## Claims

1. An ultrasonic measuring device comprising:
a mounting member configured and arranged to be mounted on a test subject;
a support unit fixed on the mounting member;
a plurality of ultrasonic element array units supported on the support unit;
a plurality of actuators operatively coupled respectively to the ultrasonic element array units, each of the actuators being configured and arranged to individually displace a corresponding one of the ultrasonic element array units with respect to the support unit; and
a control unit configured to control the ultrasonic element array units to transmit and receive ultrasonic waves, the control unit being further configured to control the actuators.

2. The ultrasonic measuring device according to claim 1, wherein
each of the actuators is configured and arranged to generate a drive force on the corresponding one of the ultrasonic element array units in a direction orthogonal to an array surface of the corresponding one of the ultrasonic element array units.

3. The ultrasonic measuring device according to claim 1 or 2, wherein
each of the actuators is configured and arranged to generate a drive force on the corresponding one of the ultrasonic element array units around at least one of an x axis and a y axis in a two dimensional coordinate system parallel to an array surface of the corresponding one of the ultrasonic element array units.

4. The ultrasonic measuring device according to claim 3, wherein
each of the actuators is configured and arranged to generate a drive force on the corresponding one of the ultrasonic element array units around both of the x axis and the y axis in the two dimensional coordinate system.

5. The ultrasonic measuring device according to any one of claims 1 to 4,
wherein
the control unit is configured to control the actuators to change a pressing pressure of the ultrasonic element array units on the test subject so that the ultrasonic element array units transmit and receive the ultrasonic waves at a first pressure value and at a second pressure value that is different from the first pressure value.

6. The ultrasonic measuring device according to any one of claims 1 to 5,
wherein
the control unit is configured to control the ultrasonic element array units to transmit and receive surface acoustic waves between adjacent ones of the ultrasonic element array units.

7. The ultrasonic measuring device according to claim 6, wherein
the control unit, when pressing the ultrasonic element array units on the test subject, is configured to control the actuators to change orientation of the ultrasonic element array units with respect to the support unit based on a distance measured by transmission and reception of the surface acoustic waves.

8. The ultrasonic measuring device according to any one of claims 1 to 7,
wherein
the control unit is configured to control one of the actuators to release a pressing pressure of the corresponding one of the ultrasonic element array units on the test subject, and to change orientation of the corresponding one of the ultrasonic element array units around at least one of an x axis and a y axis in a two dimensional coordinate system parallel to an array surface of the corresponding one of the ultrasonic element array units.

9. The ultrasonic measuring device according to claim 8, wherein
the control unit is configured to control the one of the actuators to change the orientation of the corresponding one of the ultrasonic element array units around both of the x axis and the y axis in the two dimensional coordinate system.
